(19) **Europäisches Patentamt / European Patent Office / Office européen des brevets**

(11) **EP 4 722 237 A1**

(12) **EUROPEAN PATENT APPLICATION**
published in accordance with Art. 153(4) EPC

(43) Date of publication:
08.04.2026 Bulletin 2026/15

(21) Application number: 24814199.6

(22) Date of filing: 17.05.2024

(51) International Patent Classification (IPC):
C07K 14/62 $^{(2006.01)}$     C12N 15/17 $^{(2006.01)}$
A61K 38/28 $^{(2006.01)}$     A61P 3/10 $^{(2006.01)}$

(86) International application number:
PCT/CN2024/093792

(87) International publication number:
WO 2024/244999 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 29.05.2023 CN 202310620396

(71) Applicant: Sunshine Lake Pharma Co., Ltd.
Dongguan, Guangdong 523000 (CN)

(72) Inventors:
• LIU, Guoliang
Dongguan, Guangdong 523871 (CN)
• HU, Yulong
Dongguan, Guangdong 523871 (CN)
• GUO, Linfeng
Dongguan, Guangdong 523871 (CN)
• XU, Jun
Dongguan, Guangdong 523871 (CN)
• CHEN, Li
Dongguan, Guangdong 523871 (CN)
• YE, Fengying
Dongguan, Guangdong 523871 (CN)
• ZHOU, Jian
Dongguan, Guangdong 523871 (CN)
• LI, Xiaoping
Dongguan, Guangdong 523871 (CN)
• LI, Wenjia
Dongguan, Guangdong 523871 (CN)

(74) Representative: Hoffmann Eitle
Patent- und Rechtsanwälte PartmbB
Arabellastraße 30
81925 München (DE)

Remarks:
The complete document including Reference Table(s) and the Sequence Listing(s) can be downloaded from the EPO website

(54) **METHOD FOR DETECTING BIOLOGICAL ACTIVITY OF INSULIN OR ANALOGUE THEREOF AND KIT**

(57)     The present invention provides a method and kit for detecting biological activity of insulin or analogue thereof. The detection method includes an isolated nucleic acid molecule, the nucleic acid molecule comprises: a first nucleic acid segment encoding insulin receptor B; a second nucleic acid segment encoding a STAT5b response element; and a third nucleic acid segment encoding a reporter gene; the STAT5b response element comprising a nucleic acid sequence as shown in any one of SEQ ID NO: 1~3; the first nucleic acid segment, the second nucleic acid segment, and the second and third nucleic acid segments are linked or unlinked. The detection method of the present invention has the advantages of a larger detection window value, higher accuracy, better precision, smaller variation, simpler operation, and lower detection cost.

**Description**

This application claims priority to Chinese Patent Application No. 202310620396.9 filed with the State Intellectual Property Office on May 29, 2023; the entire contents of which are incorporated herein by reference.

**FIELD OF THE INVENTION**

The present invention relates to the field of genetic engineering. Specifically, it relates to a method and a kit for detecting the biological activity of insulin or analogs thereof. More specifically, it relates to an isolated nucleic acid molecule, an expression vector, a recombinant cell, a method for detecting the activity of insulin or an analog thereof, a kit, and uses thereof.

**BACKGROUND ART**

Insulin is a crucial hormone that performs various physiological functions in the human body, such as regulating blood glucose levels and promoting glucose uptake and utilization. Detecting insulin activity can assist physicians or researchers in gaining a better understanding of an individual's insulin levels and functional status. By detecting insulin activity, diabetes can be diagnosed and monitored. Diabetic patients often experience hyperglycemia due to issues such as insufficient insulin secretion or abnormalities in insulin receptors. Detecting insulin activity helps doctors determine the type of diabetes and guides the development and adjustment of treatment plans. Furthermore, in drug development and clinical trials, detecting the activity of insulin or its analogs aids in evaluating the impact and efficacy of new drugs on insulin, thereby directing research directions and informing the selection of therapeutic approaches.

The insulin signaling pathway is closely linked to type 2 diabetes. Insulin first binds to the insulin receptor (IR) on the cell membrane. The IR is a transmembrane glycoprotein with tyrosine kinase activity. Upon binding to the IR, insulin activates its tyrosine kinase activity and phosphorylates insulin receptor substrates (IRSs), triggering a series of physiological responses within the cell. Many substrate molecules of IR have been identified, and signal transducer and activator of transcription 5b (STAT5b) is one of them. STAT5b interacts with tyrosine at position 960 of the IRβ subunit through its SH2 sequence and can be phosphorylated by the purified insulin receptor tyrosine kinase active domain. Insulin stimulates tyrosine phosphorylation of endogenous STAT5b in cells with high IR expression.

Currently, commercial kits such as HTRF (homogeneous time-resolved fluorescence) and AlphaElisa are commonly used to assess insulin activity. These assays measure insulin IR β receptor phosphorylation levels to characterize insulin's cellular bioactivity. On the one hand, commercial test kits such as HTRF and AlphaElisa are expensive and have high detection costs; on the other hand, technical means such as HTRF and AlphaElisa are relatively complicated to operate, usually requiring the addition of insulin drugs to co-incubate with cells, then adding lysis buffer for incubation and lysis, and then adding the corresponding phosphorylated antibody solution for incubation, and finally testing. Under normal circumstances, the sample volume of each step of these kits needs to be controlled to 4~8 μl, which requires a high level of operational skills of the analyst. If the sample volume is increased, the volume of reagents used in the kit must also be increased to meet the requirements of the detection method, and the detection cost will also be greatly increased, which is not conducive to the control of the company's quality control detection cost and has relatively low practicality.

Therefore, there is an urgent need in this field to develop a method for detecting insulin activity that is efficient, accurate, highly sensitive, has a wide window value, is easier to operate, and has lower detection costs.

**SUMMARY**

This application is based on the inventors' discoveries and understanding of the following facts and issues: While existing methods for detecting insulin activity can be performed using commercial kits such as HTRF (homogeneous time-resolved fluorescence) and AlphaElisa, their high cost and cumbersome detection procedures severely limit their translational applications. Therefore, the inventors designed a novel STAT5b response element sequence to construct a cell line containing a luciferase reporter gene, allowing for the detection of insulin or its analog activity in the presence of luciferase. The present detection method overcomes the high cost and low practicality of commercial kits, offering advantages such as a wider detection window, higher accuracy, improved precision, reduced variability, simpler operation, and lower detection costs.

In a first aspect of the present invention, the present invention provides an isolated nucleic acid molecule. According to the embodiment of the present invention, the isolated nucleic acid molecule comprises a first nucleic acid segment encoding insulin receptor B; a second nucleic acid segment encoding a STAT5b response element; and a third nucleic acid segment encoding a reporter gene; the STAT5b response element comprising a nucleic acid sequence as shown in any one of SEQ ID NO: 1~3, wherein the first nucleic acid segment, the second nucleic acid segment, and the second and third nucleic acid segments are linked or unlinked. Preferably, the second nucleic acid fragment is linked to the third nucleic acid fragment.

The first segment - Insulin receptor (IR-B): A transmembrane glycoprotein possessing tyrosine kinase activity. Upon binding to the IR, insulin activates its tyrosine kinase activity and phosphorylates insulin receptor substrates (IRSs), triggering a series of physiological responses within the cell. The need to overexpress the target receptor in the constructed cells is the basis for inventing the IRB phosphorylation biological activity detection of insulin and its analogs. The first segment of the present invention is not connected to the second segment and the third segment. According to the principles of molecular biology, the first segment can be independent of the second segment and the third segment, or can be connected to the second segment and the third segment before transfection and construction.

The second fragment - STAT5b response element: After the insulin receptor B is activated, the signal is transduced to the STAT5b response element and transcription activator 5b (signal transducer and activator of transcription 5b, STAT5b), which is one of the IRS. That is, after the insulin receptor B is activated, the STAT5b response element will be activated and mediate the increase in the level of STAT5b.

The third segment - reporter gene: STAT5b activated by insulin receptor tyrosine kinase (IRTK) binds to the target gene regulatory sequence with STAT5b response elements in the nucleus, thereby activating the transcription and expression of the target gene. That is, the activated second fragment STAT5b response element regulates the transcription of the reporter gene and the expression of luciferase, thereby testing the luminescence value after adding the luciferase substrate and then translating the level of luciferase to reflect the level of STAT5b and finally reflect the phosphorylation level of insulin receptor B. This reporter gene (the third fragment) needs to be directly associated with and respond to the level of STAT5b. Therefore, the second and third fragments need to be connected so that after the STAT5b response element is activated, the constructed cell reporter gene is activated as the next signal, causing the cell to transcribe and express luciferase, and then ultimately reflect the phosphorylation level of insulin receptor B, that is, reflect the IRB biological activity of insulin and its analogs.

According to an embodiment of the present invention, after the insulin receptor B in the isolated nucleic acid molecule binds to insulin, it activates the tyrosine kinase activity of the insulin receptor B and phosphorylates and activates the signal transducer and activator of transcription 5b (STAT5b), thereby causing the transcription and expression of the reporter gene. By detecting the expression level of the reporter gene, the activity of insulin or its analogue is determined. The detection method of the present invention has higher sensitivity, stronger specificity, better repeatability and higher accuracy, and is more suitable as a quality control analysis method in industrial production.

GGTACCTGAGCTCGCTAGCGCTGCTTCCGGGAAGGGCTGCTGCTTCCGGGAAGG

GCTGCTGCTTCCGGGAAGGGCT AGATCT (SEQ ID NO:1);

GGTACCTGAGCTCGCTAGCTGTGTGGACTTCTTGGAATTAAGGGACTTTTGTGTG

GACTTCTTGGAATTAAGGGACTTTTGTGTGGACTTCTTGGAATTAAGGGACTTTAGATC

T (SEQ ID NO: 2);

GGTACCTGAGCTCGCTAGCCTGATTTCCCCGAAATGACTGATTTCCCCGAAATGA

CTGATTTCCCCGAAATGA AGATCT (SEQ ID NO: 3).

According to the embodiment of the present invention, the isolated nucleic acid molecule may further include at least one of the following additional technical features:

According to the embodiment of the present invention, the STAT5b response element comprises the nucleic acid sequence as shown in SEQ ID NO:3.

According to the embodiment of the present invention, the reporter gene comprises at least one of a Lucifererase gene, a CAT gene, a YFP gene or a GFP gene. According to the embodiment of the present invention, the aforementioned reporter gene is selected due to its advantages of high detection sensitivity, simple operation, wide application range, and visual observation.

According to the embodiment of the present invention, the reporter gene is a Lucifererase gene.

According to the embodiment of the present invention, the insulin receptor protein B comprises the amino acid sequence as shown in SEQ ID NO: 4,

MGTGGRRGAAAAPLLVAVAALLLGAAGHLYPGEVCPGMDIRNNLTRLHELENCSVIE

GHLQILLMFKTRPEDFRDLSFPKLIMITDYLLLFRVYGLESLKDLFPNLTVIRGSRLFFNYA

LVIFEMVHLKELGLYNLMNITRGSVRIEKNNELCYLATIDWSRILDSVEDNYIVLNKDDNE

ECGDICPGTAKGKTNCPATVINGQFVERCWTHSHCQKVCPTICKSHGCTAEGLCCHSECL

GNCSQPDDPTKCVACRNFYLDGRCVETCPPPYYHFQDWRCVNFSFCQDLHHKCKNSRR

QGCHQYVIHNNKCIPECPSGYTMNSSNLLCTPCLGPCPKVCHLLEGEKTIDSVTSAQELR

GCTVINGSLIINIRGGNNLAAELEANLGLIEEISGYLKIRRSYALVSLSFFRKLRLIRGETLEI

GNYSFYALDNQNLRQLWDWSKHNLTITQGKLFFHYNPKLCLSEIHKMEEVSGTKGRQER

NDIALKTNGDQASCENELLKFSYIRTSFDKILLRWEPYWPPDFRDLLGFMLFYKEAPYQN

VTEFDGQDACGSNSWTVVDIDPPLRSNDPKSQNHPGWLMRGLKPWTQYAIFVKTLVTFS

DERRTYGAKSDIIYVQTDATNPSVPLDPISVSNSSSQIILKWKPPSDPNGNITHYLVFWERQ

AEDSELFELDYCLKGLKLPSRTWSPPFESEDSQKHNQSEYEDSAGECCSCPKTDSQILKEL

EESSFRKTFEDYLHNVVFVPRKTSSGTGAEDPRPSRKRRSLGDVGNVTVAVPTVAAFPNT

SSTSVPTSPEEHRPFEKVVNKESLVISGLRHFTGYRIELQACNQDTPEERCSVAAYVSART

MPEAKADDIVGPVTHEIFENNVVHLMWQEPKEPNGLIVLYEVSYRRYGDEELHLCVSRK

HFALERGCRLRGLSPGNYSVRIRATSLAGNGSWTEPTYFYVTDYLDVPSNIAKIIIGPLIFV

FLFSVVIGSIYLFLRKRQPDGPLGPLYASSNPEYLSASDVFPCSVYVPDEWEVSREKITLLR

ELGQGSFGMVYEGNARDIIKGEAETRVAVKTVNESASLRERIEFLNEASVMKGFTCHHVV

RLLGVVSKGQPTLVVMELMAHGDLKSYLRSLRPEAENNPGRPPPTLQEMIQMAAEIADG

MAYLNAKKFVHRDLAARNCMVAHDFTVKIGDFGMTRDIYETDYYRKGGKGLLPVRWM

APESLKDGVFTTSSDMWSFGVVLWEITSLAEQPYQGLSNEQVLKFVMDGGYLDQPDNC

PERVTDLMRMCWQFNPKMRPTFLEIVNLLKDDLHPSFPEVSFFHSEENKAPESEELEMEF

EDMENVPLDRSSHCQREEAGGRDGGSSLGFKRSYEEHIPYTHMNGGKKNGRILTLPRSN

PS (SEQ ID NO: 4).

According to the embodiment of the present invention, the reporter gene comprises the amino acid sequence as shown in SEQ ID NO: 5,

MEDAKNIKKGPAPFYPLEDGTAGEQLHKAMKRYALVPGTIAFTDAHIEVDITYAEYF

EMSVRLAEAMKRYGLNTNHRIVVCSENSLQFFMPVLGALFIGVAVAPANDIYNERELLNS

MGISQPTVVFVSKKGLQKILNVQKKLPIIQKIIIMDSKTDYQGFQSMYTFVTSHLPPGFNE

YDFVPESFDRDKTIALIMNSSGSTGLPKGVALPHRTACVRFSHARDPIFGNQIIPDTAILSVV

PFHHGFGMFTTLGYLICGFRVVLMYRFEEELFLRSLQDYKIQSALLVPTLFSFFAKSTLIDK

YDLSNLHEIASGGAPLSKEVGEAVAKRFHLPGIRQGYGLTETTSAILITPEGDDKPGAVGK

VVPFFEAKVVDLDTGKTLGVNQRGELCVRGPMIMSGYVNNPEATNALIDKDGWLHSGD

IAYWDEDEHFFIVDRLKSLIKYKGYQVAPAELESILLQHPNIFDAGVAGLPDDDAGELPAA

VVVLEHGKTMTEKEIVDYVASQVTTAKKLRGGVVFVDEVPKGLTGKLDARKIREILIKA

KKGGKIAVNSHGFPPEVEEQAAGTLPMSCAQESGMDRHPAACASARINV (SEQ ID NO: 5).

According to the embodiment of the present invention, the first nucleic acid fragment comprises the nucleotide sequence as shown in SEQ ID NO: 6,

ATGGGCACCGGGGGCCGGCGGGGGGCGGCGGCCGCGCCGCTGCTGGTGGCGGT

GGCCGCGCTGCTACTGGGCGCCGCGGGCCACCTGTACCCCGGAGAGGTGTGTCCCGG

CATGGATATCCGGAACAACCTCACTAGGTTGCATGAGCTGGAGAATTGCTCTGTCATC

GAAGGACACTTGCAGATACTCTTGATGTTCAAAACGAGGCCCGAAGATTTCCGAGAC

CTCAGTTTCCCCAAACTCATCATGATCACTGATTACTTGCTGCTCTTCCGGGTCTATGG

GCTCGAGAGCCTGAAGGACCTGTTCCCCAACCTCACGGTCATCCGGGGATCACGACT

GTTCTTTAACTACGCGCTGGTCATCTTCGAGATGGTTCACCTCAAGGAACTCGGCCTC

TACAACCTGATGAACATCACCCGGGGTTCTGTCCGCATCGAGAAGAACAATGAGCTCT

GTTACTTGGCCACTATCGACTGGTCCCGTATCCTGGATTCCGTGGAGGATAATTACATC

GTGTTGAACAAAGATGACAACGAGGAGTGTGGAGACATCTGTCCGGGTACCGCGAAG

GGCAAGACCAACTGCCCCGCCACCGTCATCAACGGGCAGTTTGTCGAACGATGTTGG

ACTCATAGTCACTGCCAGAAAGTTTGCCCGACCATCTGTAAGTCACACGGCTGCACCG

CCGAAGGCCTCTGTTGCCACAGCGAGTGCCTGGGCAACTGTTCTCAGCCCGACGACC

CCACCAAGTGCGTGGCCTGCCGCAACTTCTACCTGGACGGCAGGTGTGTGGAGACCT

GCCCGCCCCCGTACTACCACTTCCAGGACTGGCGCTGTGTGAACTTCAGCTTCTGCCA

GGACCTGCACCACAAATGCAAGAACTCGCGGAGGCAGGGCTGCCACCAGTACGTCAT

TCACAACAACAAGTGCATCCCTGAGTGTCCCTCCGGGTACACGATGAATTCCAGCAAC

TTGCTGTGCACCCCATGCCTGGGTCCCTGTCCCAAGGTGTGCCACCTCCTAGAAGGCG

AGAAGACCATCGACTCGGTGACGTCTGCCCAGGAGCTCCGAGGATGCACCGTCATCA

ACGGGAGTCTGATCATCAACATTCGAGGAGGCAACAATCTGGCAGCTGAGCTAGAAG

CCAACCTCGGCCTCATTGAAGAAATTTCAGGGTATCTAAAAATCCGCCGATCCTACGC

TCTGGTGTCACTTTCCTTCTTCCGGAAGTTACGTCTGATTCGAGGAGAGACCTTGGAA

ATTGGGAACTACTCCTTCTATGCCTTGGACAACCAGAACCTAAGGCAGCTCTGGGACT

GGAGCAAACACAACCTCACCATCACTCAGGGGAAACTCTTCTTCCACTATAACCCCA

AACTCTGCTTGTCAGAAATCCACAAGATGGAAGAAGTTTCAGGAACCAAGGGGCGCC

AGGAGAGAAACGACATTGCCCTGAAGACCAATGGGGACCAGGCATCCTGTGAAAATG

AGTTACTTAAATTTTCTTACATTCGGACATCTTTTGACAAGATCTTGCTGAGATGGGAG

CCGTACTGGCCCCCGACTTCCGAGACCTCTTGGGGTTCATGCTGTTCTACAAAGAGG

CCCCTTATCAGAATGTGACGGAGTTCGACGGGCAGGATGCATGTGGTTCCAACAGTTG

GACGGTGGTAGACATTGACCCACCCCTGAGGTCCAACGACCCCAAATCACAGAACCA

CCCAGGGTGGCTGATGCGGGGTCTCAAGCCCTGGACCCAGTATGCCATCTTTGTGAAG

ACCCTGGTCACCTTTTCGGATGAACGCCGGACCTATGGGGCCAAGAGTGACATCATTT

ATGTCCAGACAGATGCCACCAACCCCTCTGTGCCCTGGATCCAATCTCAGTGTCTAA

CTCATCATCCCAGATTATTCTGAAGTGGAAACCACCCTCCGACCCCAATGGCAACATC

ACCCACTACCTGGTTTTCTGGGAGAGGCAGGCGGAAGACAGTGAGCTGTTCGAGCTG

GATTATTGCCTCAAAGGGCTGAAGCTGCCCTCGAGGACCTGGTCTCCACCATTCGAGT

CTGAAGATTCTCAGAAGCACAACCAGAGTGAGTATGAGGATTCGGCCGGCGAATGCT

GCTCCTGTCCAAAGACAGACTCTCAGATCCTGAAGGAGCTGGAGGAGTCCTCGTTTA

GGAAGACGTTTGAGGATTACCTGCACAACGTGGTTTTCGTCCCCAGAAAAACCTCTTC

AGGCACTGGTGCCGAGGACCCTAGGCCATCTCGGAAACGCAGGTCCCTTGGCGATGT

TGGGAATGTGACGGTGGCCGTGCCCACGGTGGCAGCTTTCCCCAACACTTCCTCGAC

CAGCGTGCCCACGAGTCCGGAGGAGCACAGGCCTTTTGAGAAGGTGGTGAACAAGG

AGTCGCTGGTCATCTCCGGCTTGCGACACTTCACGGGCTATCGCATCGAGCTGCAGGC

TTGCAACCAGGACACCCCTGAGGAACGGTGCAGTGTGGCAGCCTACGTCAGTGCGAG

GACCATGCCTGAAGCCAAGGCTGATGACATTGTTGGCCCTGTGACGCATGAAATCTTT

GAGAACAACGTCGTCCACTTGATGTGGCAGGAGCCGAAGGAGCCCAATGGTCTGATC

GTGCTGTATGAAGTGAGTTATCGGCGATATGGTGATGAGGAGCTGCATCTCTGCGTCTC

CCGCAAGCACTTCGCTCTGGAACGGGGCTGCAGGCTGCGTGGGCTGTCACCGGGGA

ACTACAGCGTGCGAATCCGGGCCACCTCCCTTGCGGGCAACGGCTCTTGGACGGAAC

CCACCTATTCTACGTGACAGACTATTTAGACGTCCCGTCAAATATTGCAAAAATTATC

ATCGGCCCCCTCATCTTTGTCTTTCTCTTCAGTGTTGTGATTGGAAGTATTTATCTATTC

CTGAGAAAGAGGCAGCCAGATGGGCCGCTGGGACCGCTTTACGCTTCTTCAAACCCT

GAGTATCTCAGTGCCAGTGATGTGTTTCCATGCTCTGTGTACGTGCCGGACGAGTGGG

AGGTGTCTCGAGAGAAGATCACCCTCCTTCGAGAGCTGGGGCAGGGCTCCTTCGGCA

TGGTGTATGAGGGCAATGCCAGGGACATCATCAAGGGTGAGGCAGAGACCCGCGTGG

CGGTGAAGACGGTCAACGAGTCAGCCAGTCTCCGAGAGCGGATTGAGTTCCTCAATG

AGGCCTCGGTCATGAAGGGCTTCACCTGCCATCACGTGGTGCGCCTCCTGGGAGTGG

TGTCCAAGGGCCAGCCCACGCTGGTGGTGATGGAGCTGATGGCTCACGGAGACCTGA

AGAGCTACCTCCGTTCTCTGCGGCCAGAGGCTGAGAATAATCCTGGCCGCCCTCCCCC

TACCCTTCAAGAGATGATTCAGATGGCGGCAGAGATTGCTGACGGGATGGCCTACCTG

AACGCCAAGAAGTTTGTGCATCGGGACCTGGCAGCGAGAAACTGCATGGTCGCCCAT

GATTTACTGTCAAAATTGGAGACTTTGGAATGACCAGAGACATCTATGAAACGGATT

ACTACCGGAAAGGGGGCAAGGGTCTGCTCCCTGTACGGTGGATGGCACCGGAGTCCC

TGAAGGATGGGGTCTTCACCACTTCTTCTGACATGTGGTCCTTTGGCGTGGTCCTTTG

GGAAATCACCAGCTTGGCAGAACAGCCTTACCAAGGCCTGTCTAATGAACAGGTGTT

GAAATTTGTCATGGATGGAGGGTATCTGGATCAACCCGACAACTGTCCAGAGAGAGTC

ACTGACCTCATGCGCATGTGCTGGCAATTCAACCCCAAGATGAGGCCAACCTTCCTGG

AGATTGTCAACCTGCTCAAGGACGACCTGCACCCCAGCTTTCCAGAGGTGTCGTTCTT

CCACAGCGAGGAGAACAAGGCTCCCGAGAGTGAGGAGCTGGAGATGGAGTTTGAGG

ACATGGAGAATGTGCCCCTGGACCGTTCCTCGCACTGTCAGAGGGAGGAGGCGGGGG

GCCGGGATGGAGGGTCCTCGCTGGGTTTCAAGCGGAGCTACGAGGAACACATCCCTT

ACACACACATGAACGGAGGCAAGAAAAACGGGCGGATTCTGACCTTGCCTCGGTCCA

ATCCTTCCTAA (SEQ ID NO: 6).

According to the embodiment of the present invention, the third nucleic acid fragment comprises the nucleotide sequence as shown in SEQ ID NO: 7,

ATGGAAGATGCCAAAAACATTAAGAAGGGCCCAGCGCCATTCTACCCACTCGAA

GACGGGACCGCCGGCGAGCAGCTGCACAAAGCCATGAAGCGCTACGCCCTGGTGCC

CGGCACCATCGCCTTTACCGACGCACATATCGAGGTGGACATTACCTACGCCGAGTAC

TTCGAGATGAGCGTTCGGCTGGCAGAAGCTATGAAGCGCTATGGGCTGAATACAAACC

ATCGGATCGTGGTGTGCAGCGAGAATAGCTTGCAGTTCTTCATGCCCGTGTTGGGTGC

CCTGTTCATCGGTGTGGCTGTGGCCCCAGCTAACGACATCTACAACGAGCGCGAGCTG

CTGAACAGCATGGGCATCAGCCAGCCCACCGTCGTATTCGTGAGCAAGAAAGGGCTG

CAAAAGATCCTCAACGTGCAAAAGAAGCTACCGATCATACAAAAGATCATCATCATGG

ATAGCAAGACCGACTACCAGGGCTTCCAAAGCATGTACACCTTCGTGACTTCCCATTT

GCCACCCGGCTTCAACGAGTACGACTTCGTGCCCGAGAGCTTCGACCGGGACAAAAC

CATCGCCCTGATCATGAACAGTAGTGGCAGTACCGGATTGCCCAAGGGCGTAGCCCTA

CCGCACCGCACCGCTTGTGTCCGATTCAGTCATGCCCGCGACCCCATCTTCGGCAACC

AGATCATCCCCGACACCGCTATCCTCAGCGTGGTGCCATTTCACCACGGCTTCGGCAT

GTTCACCACGCTGGGCTACTTGATCTGCGGCTTTCGGGTCGTGCTCATGTACCGCTTC

GAGGAGGAGCTATTCTTGCGCAGCTTGCAAGACTATAAGATTCAATCTGCCCTGCTGG

TGCCCACACTATTTAGCTTCTTCGCTAAGAGCACTCTCATCGACAAGTACGACCTAAG

CAACTTGCACGAGATCGCCAGCGGCGGGGCGCCGCTCAGCAAGGAGGTAGGTGAGG

CCGTGGCCAAACGCTTCCACCTACCAGGCATCCGCCAGGGCTACGGCCTGACAGAAA

CAACCAGCGCCATTCTGATCACCCCCGAAGGGGACGACAAGCCTGGCGCAGTAGGCA

AGGTGGTGCCCTTCTTCGAGGCTAAGGTGGTGGACTTGGACACCGGTAAGACACTGG

GTGTGAACCAGCGCGGCGAGCTGTGCGTCCGTGGCCCCATGATCATGAGCGGCTACG

TTAACAACCCCGAGGCTACAAACGCTCTCATCGACAAGGACGGCTGGCTGCACAGCG

GCGACATCGCCTACTGGGACGAGGACGAGCACTTCTTCATCGTGGACCGGCTGAAGA

GCCTGATCAAATACAAGGGCTACCAGGTAGCCCCAGCCGAACTGGAGAGCATCCTGC

TGCAACACCCCAACATCTTCGACGCCGGGGTCGCCGGCCTGCCCGACGACGATGCCG

GCGAGCTGCCCGCCGCAGTCGTCGTGCTGGAACACGGTAAAACCATGACCGAGAAG

GAGATCGTGGACTATGTGGCCAGCCAGGTTACAACCGCCAAGAAGCTGCGCGGTGGT

GTTGTGTTCGTGGACGAGGTGCCTAAAGGACTGACCGGCAAGTTGGACGCCCGCAAG

ATCCGCGAGATTCTCATTAAGGCCAAGAAGGGCGGCAAGATCGCCGTGAATTCTCACG

GCTTCCCTCCCGAGGTGGAGGAGCAGGCCGCCGGCACCCTGCCCATGAGCTGCGCCC

AGGAGAGCGGCATGGATAGACACCCTGCTGCTTGCGCCAGCGCCAGGATCAACGTCT

AA (SEQ ID NO: 7).

It should be noted that the reporter gene can be replaced based on experimental requirements, or can be obtained by purchasing a plasmid containing the reporter gene.

In the second aspect of the present invention, the present invention provides an expression vector. According to the embodiment of the present invention, the expression vector carries at least any one of the nucleic acid fragments or nucleic acid molecules described in the first aspect of the present invention. According to the embodiment of the present invention, the expression vector can efficiently express the nucleic acid molecule in a suitable recipient cell, and the nucleic acid molecule has stronger specificity.

According to the embodiment of the present invention, the expression vector may further include at least one of the following additional technical features:

According to the embodiment of the present invention, the expression vector is a non-pathogenic viral vector.

According to the embodiment of the present invention, the non-pathogenic virus is selected from at least one of a retrovirus, a lentivirus and an adenovirus-associated virus.

In the third aspect of the present invention, the present invention provides a recombinant cell. According to the embodiment of the present invention, the recombinant cell carries the nucleic acid molecule described in the first aspect of the present invention or the expression vector described in the second aspect of the present invention.

According to some specific embodiments of the present invention, the recombinant cells can efficiently and massively express nucleic acid molecules under appropriate conditions. The nucleic acid molecules have stronger recognition specificity for insulin or its analogs. Efficient detection can be achieved with a smaller sample volume, lower cost, higher detection accuracy and sensitivity, and a larger window value, which is of great significance for the industrial production of detection of insulin or its analogs.

# EP 4 722 237 A1

According to the embodiment of the present invention, the recombinant cell may further include at least one of the following additional technical features:

According to the embodiment of the present invention, the recombinant cell is selected from at least one of CRFK, HEK293FT, HEK293T, HEK293 or BHK cells.

According to the embodiment of the present invention, the recombinant cell is a HEK293 cell. In the fourth aspect of the present invention, the present invention provides a method for detecting the biological activity of insulin or analogs thereof. According to the embodiment of the present invention, the method includes: contacting a sample of insulin or its analogues to be tested with the recombinant cells described in the third aspect of the present invention; performing luminescence signal detection processing on the contact treatment product, and calculating the biological activity of insulin or its analogues based on the luminescence signal.

According to the specific embodiment of the present invention, the method for detecting insulin or its analogs developed by the inventors has the advantages of a larger detection window value, higher accuracy, better precision, smaller variation, simpler operation, and lower detection cost.

According to the embodiment of the present invention, the method may further include at least one of the following additional technical features:

According to the embodiment of the present invention, after the contact treatment and before the detection treatment, an incubation treatment is further included. The incubation treatment involves incubating the contacted product in a 37 °C, 5% $CO_2$ incubator for a predetermined period of time, preferably 10-20 h, more preferably 15 h.

It should be noted that, depending on the choice of reporter gene, after incubation, luciferase substrate needs to be added to the incubation product before fluorescence signal detection can be performed. If the reporter gene selected allows for direct fluorescence excitation, the addition of a luciferase substrate is not necessary. The aforementioned luciferase substrate can be generally selected based on the experimental situation.

According to the embodiment of the present invention, the volume of the sample to be tested is 18-24 μl, preferably 20 μl.

In the fifth aspect of the present invention, the present invention provides a kit. According to the embodiment of the present invention, the kit includes: the nucleic acid molecule described in the first aspect of the present invention, the expression vector described in the second aspect of the present invention, or the recombinant cell described in the third aspect of the present invention. The kit of the present invention can detect insulin or its analogs quickly and accurately, has lower cost, and can be widely used in scientific research and medical clinical applications.

According to the embodiment of the present invention, the kit may further include at least one of the following additional technical features:

According to the embodiment of the present invention, the kit further includes an activity curve or instructions of a standard substance of insulin or its analogs.

In the sixth aspect of the present invention, the present invention proposes a use of the nucleic acid molecule described in the first aspect of the present invention, the expression vector described in the second aspect of the present invention, the recombinant cell described in the third aspect of the present invention, or the kit described in the fourth aspect of the present invention in detecting the biological activity of insulin or its analogs. According to the embodiment of the present invention, the kit for detecting the activity of insulin or its analogs has the advantages of a larger detection window value, high accuracy, ease of use, and good economy.

Additional aspects and advantages of the present invention will be set forth in part in the description which follows and, in part, will be obvious from the description which follows, or may be learned by practice of the present invention.

## Description of the drawings

The above and/or additional aspects and advantages of the present invention will become apparent and readily understood from the following description of the embodiments with reference to the accompanying drawings, in which:

Figure 1 is a plasmid map of the Luciferase reporter gene containing the CRE response element according to Example 1 of the present invention;

Figure 2 is the performance evaluation results of cell lines corresponding to different STAT5b response element sequences according to Example 1 of the present invention;

Figure 3 is the functional evaluation results of monoclonal screened cell lines according to Example 1 of the present invention;

Figure 4 is the specificity validation results of the Luciferase reporter gene detection method according to Example 2 of the present invention;

Figure 5 is the linearity validation results of the Luciferase reporter gene detection method according to Example 2 of the present invention;

Figure 6 is the specificity validation results of the HTRF assay according to Example 3 of the present invention;

11

Figure 7 is the linearity validation results of the HTRF assay according to Example 3 of the present invention;

Figure 8 is the activity detection results of ultra-long-acting insulin by the Luciferase reporter gene detection method according to Example 4 of the present invention;

Figure 9 is the activity detection results of different insulin preparations by the Luciferase reporter gene detection method according to Example 4 of the present invention;

Figure 10 is the activity detection results of the Degludec/Aspart combination formulation by the Luciferase reporter gene detection method according to Example 4 of the present invention;

Figure 11 is the activity detection results of Insulin Glargine Injection (Sanofi) by the Luciferase reporter gene detection method according to Example 4 of the present invention;

Figure 12 is the activity detection results of Insulin Glargine Injection (HEC) by the Luciferase reporter gene detection method according to Example 4 of the present invention.

## EXAMPLES

Examples of the present invention are described in detail below, examples of which are illustrated in the accompanying drawings. The examples described below with reference to the accompanying drawings are exemplary and are intended to explain the present invention, but should not be construed as limiting the present invention.

## Definitions and Explanations

Furthermore, the terms "first" and "second" are used for descriptive purposes only and should not be construed as indicating or implying relative importance or implicitly specifying the number of technical features being identified. Thus, features specified as "first" or "second" may explicitly or implicitly include at least one such feature. In the present description, "plurality" means at least two, such as two, three, etc., unless otherwise specifically defined.

The endpoints of the ranges and any values disclosed herein are not limited to the exact ranges or values; such ranges or values should be understood to encompass values approximating these ranges or values. For numerical ranges, the values between the endpoints of each range, between the endpoints of each range and individual points, and between individual points can be combined to generate one or more new numerical ranges, and these numerical ranges should be considered specifically disclosed herein.

In this document, unless otherwise specified, IR stands for insulin receptor, a transmembrane glycoprotein with tyrosine kinase activity. The insulin receptor IR is divided into two subtypes: IRA and IRB. Among, IRB is the primary receptor for insulin-induced blood glucose regulation. Insulin stimulates cell lines bearing the insulin receptor, and measuring the tyrosine kinase activity of the IRB receptor indirectly reflects the biological activity of insulin.

## Isolated nucleic acid molecule

In one aspect of the present invention, the present invention provides an isolated nucleic acid molecule. According to the embodiment of the present invention, the nucleic acid molecule includes sequences for expressing insulin receptor B, a STAT5b response element and a reporter gene. The aforementioned insulin receptor B, STAT5b response element and reporter gene sequence are connected.

## Expression vector

In another aspect of the present invention, the present invention provides an expression vector. According to the embodiment of the present invention, the expression vector carries the aforementioned isolated nucleic acid molecule.

## Recombinant cell

In another aspect of the present invention, the present invention provides an expression vector. According to the embodiment of the present invention, the recombinant cell carries the aforementioned nucleic acid molecule or expression vector.

## Method for detecting the activity of insulin or its analogs

In one aspect of the present invention, the present invention provides a method for detecting the activity of insulin or analogs thereof. According to the embodiment of the present invention, the method includes contacting an insulin sample with the aforementioned recombinant cell; performing luminescence signal detection on the contact product, and determining the activity of insulin or its analog based on the luminescence signal.

Specifically, for ease of understanding, the technical solution of this application is explained and illustrated in detail below:

1) Preparation of Reference and Test Solution

① 0.6 mM insulin glargine reference solution: An appropriate amount of insulin glargine reference was weighed, dissolved completely in 0.01 M HCL and diluted to 0.6 mM, and mixed thoroughly.

It should be noted that the reference solution can be selected from a commercially available standard solution or a solution prepared from pure reagents.

② 0.6 mM Insulin Glargine Test Solution: An appropriate amount of insulin glargine injection solution was taken.

③ 15000 nM Reference/Test Solution Stock Solution: 780 μl of 0.01 M hydrochloric acid solution was added to 20 μl of a 0.6 mM insulin glargine reference/test solution and mixed thoroughly.

④ 1000 nM control/test solution: 780 μl of DMEM medium containing 1% FBS (fetal bovine serum) was added to 20 μl of a 15000 nM reference/test solution.

⑤ Serial dilutions of control/test compounds: Dilutions were performed in a 96-well plate according to the method shown in Table 1.

Table 1:

| No. | Concentration of reference/test compound serial dilutions (nM) | Dilution method |
|---|---|---|
| R/T-1 | 1000 | 250 μl of the control/test solution was added and mixed. |
| R/T-2 | 625 | 90 μl of the blank solution was added to 150 μl of R/T-1 solution and mixed. |
| R/T-3 | 390.625 | 90 μl of the blank solution was added to 150 μl of R/T-2 solution and mixed. |
| R/T-4 | 244.14063 | 90 μl of the blank solution was added to 150 μl of R/T-3 solution and mixed. |
| R/T-5 | 152.58789 | 90 μl of the blank solution was added to 150 μl of R/T-4 solution and mixed. |
| R/T-6 | 95.367432 | 90 μl of the blank solution was added to 150 μl of R/T-5 solution and mixed. |
| R/T-7 | 59.604645 | 90 μl of the blank solution was added to 150 μl of R/T-6 solution and mixed. |
| R/T-8 | 37.252903 | 90 μl of the blank solution was added to 150 μl of R/T-7 solution and mixed. |
| R/T-9 | 23.283064 | 90 μl of the blank solution was added to 150 μl of R/T-8 solution and mixed. |
| R/T-10 | 7.7610215 | 160 μl of the blank solution was added to 80 μl of R/T-9 solution and mixed. |
| R/T-11 | 2.5870072 | 160 μl of the blank solution was added to 80 μl of R/T-10 solution and mixed. |
| Note: R represents the control group; T represents the test group. | | |

2) Preparation of Cell Suspension

The culture flask was taken out of the incubator, the culture medium was discarded, and 2 ml of DPBS (sterile saline) buffer was added and gently washed twice. 1 ml of DPBS and 1 ml of trypsin solution were slowly added along the cell-free side of the flask to digest the cells for approximately 2 min. When cytoplasm retraction, cell gap enlargement or a few cells were found to have fallen off, the culture flask was gently shaken to make all the cells fall off, 3 ml of complete culture medium was added to the culture flask, and the cells were dispersed by gently pipetting. The cell suspension was transferred to a centrifuge tube with a pipette, centrifuged at 900 rpm for 5 min, the supernatant was poured out, and 1 ml of DMEM medium containing 1% FBS was added to resuspend the cells. The cells were gently pipetted with a pipette to make a single suspension. 10 μl was taken out and transferred to a 0.2 ml centrifuge tube. 10 μl of trypan blue solution was added to the tube and pipetted evenly. 10 μl of the mixture was taken out and transferred to a cell counting plate to calculate the density of viable cells.

The cell suspension density was adjusted to 500000 cells/ml using DMEM medium containing 1% FBS. 20 μl of the cell suspension was added to each well of a 384-deep-well plate using a 12-channel dispensable pipette, resulting in a cell population of 10000 cells/well.

3) Co-incubation

The 2-fold concentration of the control (R)/test (T) solution was added to the corresponding wells using a pipette along the wall, 20 μl per well, and three replicates were added for each concentration point.

After the above solutions were added, the 384-well plate was covered with a transparent sealing film and placed in a 37 °C, 5% $CO_2$ incubator for 15 h.

4) Detection of Luminescence Signals

After 15 h of incubation, the 384-well plate was removed from the incubator and allowed to stand at room temperature for approximately 5 min. Then, 30 μl of luciferase substrate was quickly added to each well using a pipette against the wall. After 3 minutes, the luminescence signal was detected in the LUM mode on a multi-functional microplate reader.

5) Calculation

With log (actual compound concentration) as the horizontal axis and bioluminescence value as the vertical axis, a four-parameter fit was performed using GraphPad Prism software. The specific formula is as follows:

$$Y = A + \frac{D - A}{1 + 10^{\wedge}[(LogEC_{50} - X) \times B]}$$

wherein:

Y is the bioluminescence value of each well;
X is the log (compound concentration) value, nmol/L;
A is the estimated asymptote below the curve (i.e., bottom value);
D is the estimated asymptote above the curve (i.e., top value);
B is the slope of the curve (i.e., Hill Slope value);
$EC_{50}$ is the median effect concentration, nmol/L.

The relative biological activity of the test solution was calculated according to the following formula:

$$A = (EC_{50R}/EC_{50S}) \times 100\%$$

wherein:

A is the relative bioactivity of the test article, %;
$EC_{50R}$ is the median effect concentration of the reference solution calculated from the curve, nmol/L;
$EC_{50S}$ is the median effect concentration of the test article solution calculated from the curve, nmol/L.

The examples will be described in detail below. If no specific techniques or conditions are specified in the examples, the techniques or conditions described in the literature in the art or the product instructions shall be followed. All reagents and instruments used without manufacturer indication are commercially available conventional products.

It should be noted that, in the present application, the method for detecting the activity of insulin or its analogs has the same meaning as the method for detecting the luciferase reporter gene.

**Example 1: Construction of a luciferase reporter gene cell line**

1.1 Construction of Reporter Gene Plasmids

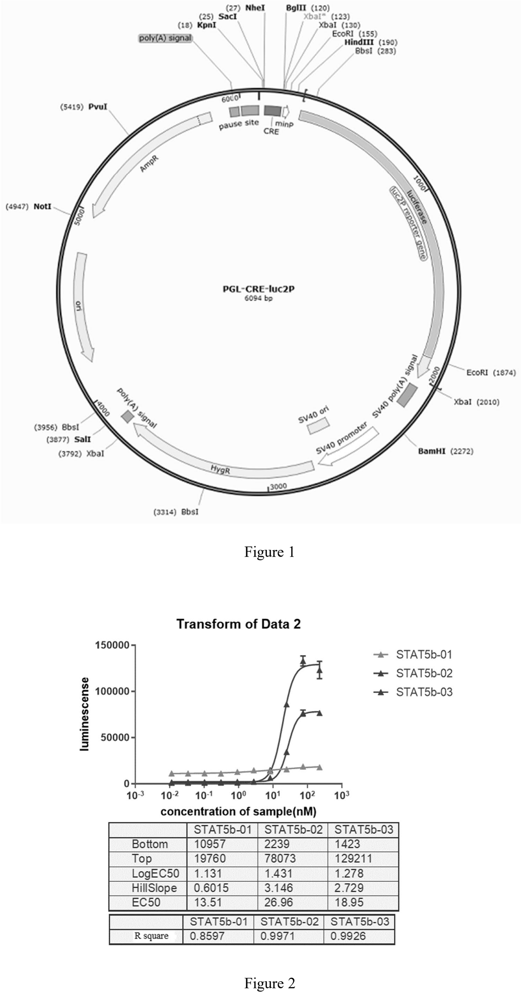

The present invention constructed three STAB5b response elements, each having the nucleic acid sequences as shown in SEQ ID NO: 1~3. The CRE response element in the Luciferase reporter gene plasmid (Figure 1) was replaced with a STAB5b response element (Guangzhou IGE Biotechnology Co., Ltd.), resulting in three Luciferase reporter gene plasmids containing the STAT5b response element (labeled STAB5b-01-Luciferase, STAB5b-02-Luciferase, and STAB5b-03-Luciferase, respectively, according to SEQ ID NO: 1~3).

1.2 Construction of cell line

1.2.1 The HEK293-IRB monoclonal cell line that had been transferred into the insulin receptor IRB was removed from the liquid nitrogen tank and cultured at 37 °C, 5% $CO_2$ (the culture conditions below are all 37 °C, 5% $CO_2$) in DMEM complete medium containing 10.0% FBS and 1% double-streptomycin (penicillin/streptomycin). After recovery for 3 days, $0.4 * 10^6$ cells/well were seeded into 6-well plates and incubated in an incubator for approximately 48 h before use.

1.2.2 The punctured bacteria containing the three reporter gene plasmids, STAT5b-01-Luciferase, STAT5b-02-Luciferase and STAT5b-03-Luciferase, were inoculated into LB liquid medium (containing 100 $\mu$g/mL ampicillin) and cultured with shaking at 250 rpm at 37 °C overnight. The plasmids were extracted using a plasmid extraction kit (Thermo). The constructed recombinant plasmids were linearized with Pvu I endonuclease, and the linearized products were recovered. The linearized recombinant expression plasmids were stably transfected into HEK293-IRB cells using Lipofectamine LP3000 transfection. Three stable transfected cell lines, HEK293/IRB-STAT5b-01-Luciferase, HEK293/IRB-STAT5b-02-Luciferase, and HEK293/IRB-STAT5b-03-Luciferase, were obtained by hygromycin B pressure selection (final concentration 300 $\mu$g/mL). After luciferase assay, two hybrid cell lines, HEK293/IRB-STAT5b-02-Luciferase and HEK293/IRB-STAT5b-03-Luciferase, were selected for subsequent cloning screening.

1.2.3 The two mixed cell lines after pressure screening were plated in 96-well plates at 0.30 cells/well for limiting dilution monoclonal screening. The cells were cultured in 96-well plates for 15 days, and single cell clusters were selected under microscopic examination. 48 cell clones were selected and subcultured to 24-well plates for 5 days. 35 cell clones with normal cell growth in the 24-well plates were selected under microscopic examination and subcultured at a ratio of 1:2. Each cell clone was subcultured in 2 wells, with cells from one well used for subculture and cells from the other well used for analysis and detection of the screening results. The cells in the 24-well plate were cultured in a carbon dioxide incubator for 6 days. Based on the analysis and test results, 6 cell clones were selected for expansion and culture and subcultured into T25 culture flasks, which were labeled as: 001#clone, 002#clone, 003#clone, 004#clone, 005#clone and 006#clone. The cells in T25 culture flasks were cultured in a carbon dioxide incubator for 3 days and then passaged at a ratio of 1:2. Each cell clone was passaged through two T25 flasks, with one flask of cells used for passage and the other flask of cells sent for analysis for further testing and screening. According to the analysis of activity test results, 001#clone was finally selected as the best clone. 001#clone was derived from the HEK293/IRB-STAT5b-03-Luciferase mixed cell line.

1.3 Evaluation of cell line function

The results are shown in Figure 2. It can be seen that the cell lines corresponding to STAT5b-02-Luciferase and STAT5b-03-Luciferase have a higher response to insulin stimulation and a large detection window value (the signal-to-noise ratio can be as high as more than 90 times), which meets the requirements of biological activity analysis. Among them, the STAT5b-03 sequence element had a greater response and was selected as the preferred element for further monocloning to verify the cell construction effect.

1.3.1 Functional evaluation of cell lines screened for monoclonal selection

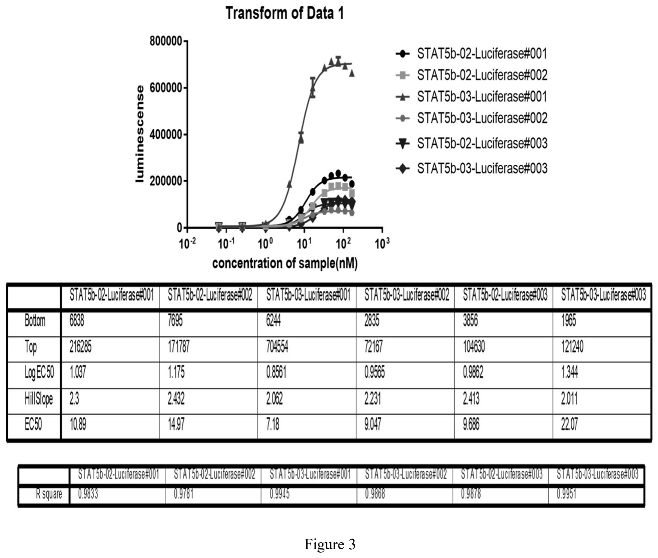

The results are shown in Figure 3. The HEK293/IRB-STAT5b-03-Luciferase#001 clone was the most sensitive to insulin stimulation and had the largest detection window (the signal-to-noise ratio was as high as 100 times or more). The most preferred cell line 001#clone was obtained, and the corresponding STAT5b-03 sequence element was the most preferred sequence element, and the next step of analysis method development was carried out.

**Example 2: Methodological verification of luciferase reporter gene**

2.1 Specificity Verification

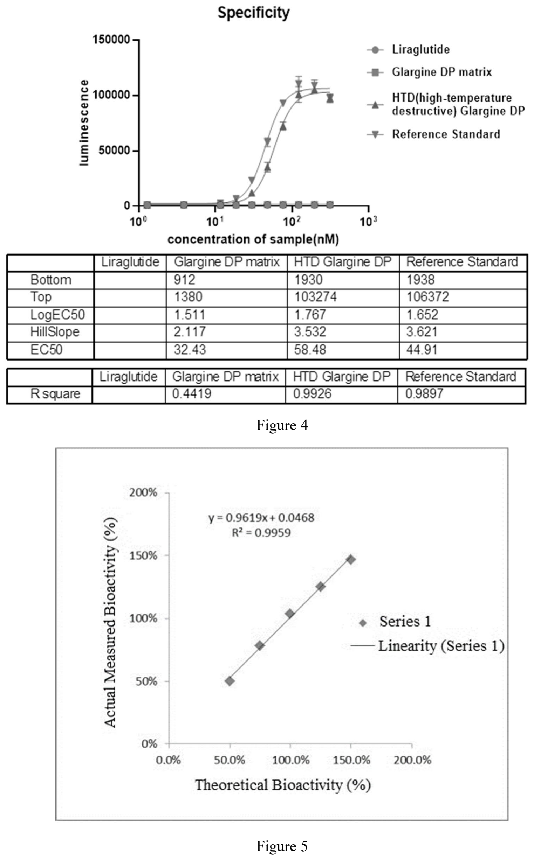

As shown in Figure 4, there was no obvious dose-effect relationship after stimulation of cells with the liraglutide (non-target drug) group and the blank solvent group of insulin glargine injection (pure solvent without the addition of insulin glargine API). However, there was a significant dose-effect relationship (the relationship between the input amount and the effect produced) after stimulation of cells with the reference standard group of insulin glargine injection and the high-temperature destruction solution group of insulin glargine. The linear relationship was good, with $R^2$ greater than 0.98. The $EC_{50}$ value of the high-temperature destruction solution group (58.48 nM) was significantly greater than the $EC_{50}$ value of the reference group (44.91 nM). In other words, the method can sensitively detect that the biological activity of the insulin glargine injection has decreased to a certain extent after being destroyed by high temperature. In summary, this analytical method has better specificity.

2.2 Verification of precision (repeatability and intermediate precision)

Different analysts tested the same batch of test samples (prepared in 6 portions in parallel) and reference materials using the above-mentioned assay method on different dates. The statistical results are shown in Tables 2~4:

Table 2:

| Personnel | Parameters / Test Solution | | $EC_{50}$(nM) | Relative biological activity | GCV value (n=6) |
|---|---|---|---|---|---|
| 1 | Test solution-1 | R | 68.89 | 106% | 1.24% |
| | | T | 65.19 | | |
| | Test solution-2 | R | 69.10 | 105% | |
| | | T | 65.99 | | |
| | Test solution-3 | R | 68.50 | 107% | |
| | | T | 64.04 | | |
| | Test solution-4 | R | 64.05 | 103% | |
| | | T | 62.11 | | |
| | Test solution-5 | R | 65.05 | 104% | |
| | | T | 62.33 | | |
| | Test solution-6 | R | 65.22 | 105% | |
| | | T | 62.18 | | |

Table 3:

| Personnel | Parameters / Test Solution | | $EC_{50}$(nM) | Relative biological activity | GCV value (n=6) |
|---|---|---|---|---|---|
| 2 | Test solution-1 | R | 79.91 | 95% | 4.84% |
| | | T | 84.08 | | |
| | Test solution-2 | R | 81.04 | 101% | |
| | | T | 79.97 | | |
| | Test solution-3 | R | 81.45 | 106% | |
| | | T | 76.85 | | |
| | Test solution-4 | R | 45.31 | 102% | |
| | | T | 44.45 | | |
| | Test solution-5 | R | 46.26 | 108% | |
| | | T | 42.93 | | |
| | Test solution-6 | R | 45.68 | 107% | |
| | | T | 42.70 | | |

Table 4:

| Parameters / Test Solution | Relative biological activity (%) | | GCV value (n=12) |
|---|---|---|---|
| | Analyst 1 | Analyst 2 | |
| Test solution-1 | 106% | 95% | |
| Test solution-2 | 105% | 101% | |
| Test solution-3 | 107% | 106% | 3.48% |
| Test solution-4 | 103% | 102% | |
| Test solution-5 | 104% | 108% | |
| Test solution-6 | 105% | 107% | |

Through analysis, it can be seen that different analysts used this method to conduct parallel tests on 6 test samples on different days. The individual GCV values were 1.24% and 4.84% respectively. The GCV value of 12 samples was 3.48%, indicating that this analytical methodology has good repeatability, small variability and high precision.

2.3 Verification of linearity and accuracy

Two different analysts prepared linear solutions (also known as accuracy solutions) of insulin glargine at potency levels of 50%, 75%, 100%, 125% and 150% on different dates and tested them according to the above assay method. The statistical results are shown in the following table:

Table 5:

| | Number of experiments | Theoretical relative biological activity | 50% | 75% | 100% | 125% | 150% |
|---|---|---|---|---|---|---|---|
| Analyst 1 | 1 | Measured relative biological activity | 49% | 81% | 105% | 126% | 144% |
| | 2 | | 55% | 79% | 105% | 130% | 149% |
| Analyst 2 | 1 | | 51% | 76% | 103% | 125% | 146% |
| | 2 | | 44% | 78% | 102% | 120% | 148% |
| | Average relative biological activity | | 50% | 78% | 104% | 125% | 147% |

Table 6:

| | Number of experiments | Theoretical relative biological activity | 50% | 75% | 100% | 125% | 150% |
|---|---|---|---|---|---|---|---|
| Analyst 1 | 1 | Recovery rate | 99% | 107% | 105% | 101% | 96% |
| | 2 | | 111% | 106% | 105% | 104% | 99% |
| Analyst 2 | 1 | | 103% | 102% | 103% | 100% | 97% |
| | 2 | | 88% | 104% | 102% | 96% | 99% |
| | Average recovery rate | | 100% | 105% | 104% | 100% | 98% |

A joint analysis of Tables 5, 6 and Figure 5 shows that this analytical method has a good linear relationship within the detection range of 50%~150% ($R^2 = 0.9959$) and high accuracy (the recovery rate of the accuracy solution at each titer level is between 88%~107%).

**Example 3: Comparison of different detection methods**

According to the embodiments of the present invention, the method for detecting insulin or its analogs according to the present invention is shown to have higher superiority by comparing its performance and cost with commercially available kits.

3.1 Cost Comparison

By comparing the market price with the cost of the method of the present invention (Table 7), the luciferase reporter gene analysis method for detecting the biological activity of insulin glargine injection developed by the present invention has obvious cost advantages over the technical means of commercial kits such as HTRF and AlphaElisa.

Table 7:

| Technical means | The market price of the kit/RMB | Test cost for 96-well plate | Test cost for 384-well plate |
|---|---|---|---|
| HTRF (Cisbio) | ¥138996 (10000 test packs) | About ¥21384 | About ¥5346 |
| AlphaElisa (PE) | ¥150023 (10000 test packs) | About ¥28850 | About ¥5770 |
| Luciferase reporter gene assay | ¥4200 (100 ml packs) | About ¥646 | About ¥323 |

3.2 Specificity comparison

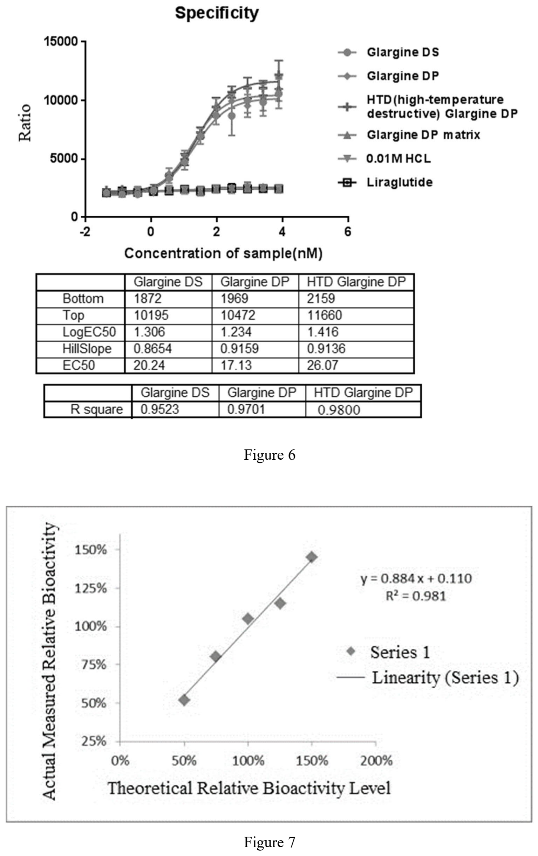

The specificity of the HTRF kit assay was similar to that of the reporter gene assay described above. It showed no response to non-target drugs (specific solution group, i.e., liraglutide), blank solvents, hydrochloric acid, and other solutions. Furthermore, it was able to detect the decrease in the biological activity of drugs destroyed by high temperature (Figure 6).

3.3 Precision comparison (repeatability and intermediate precision)

Two different analysts used the same batch of test products (prepared in 6 parallel portions) and reference products to perform tests using the HTRF kit method on different dates. The statistical results are shown in Tables 8 to 10.

Table 8:

| Personnel | Parameters / Test Solution | | $EC_{50}$(nM) | Relative biological activity | GCV value (n=6) |
|---|---|---|---|---|---|
| 1 | Test solution-1 | R | 34 | 111% | 11.60% |
| | | T | 30.55 | | |
| | Test solution-2 | R | 34.47 | 127% | |
| | | T | 27.08 | | |
| | Test solution-3 | R | 33.94 | 105% | |
| | | T | 32.36 | | |
| | Test solution-4 | R | 33.76 | 92% | |
| | | T | 36.65 | | |
| | Test solution-5 | R | 34.96 | 104% | |
| | | T | 33.77 | | |
| | Test solution-6 | R | 36.61 | 116% | |
| | | T | 31.58 | | |

Table 9:

| Personnel | Parameters / Test Solution | EC$_{50}$(nM) | | Relative biological activity | GCV value (n=6) |
|---|---|---|---|---|---|
| 2 | Test solution-1 | R | 30.35 | 96% | 18.00% |
| | | T | 31.77 | | |
| | Test solution-2 | R | 25.71 | 101% | |
| | | T | 25.53 | | |
| | Test solution-3 | R | 27.49 | 124% | |
| | | T | 22.11 | | |
| | Test solution-4 | R | 23.42 | 76% | |
| | | T | 30.9 | | |
| | Test solution-5 | R | 29.81 | 112% | |
| | | T | 26.53 | | |
| | Test solution-6 | R | 29.4 | 97% | |
| | | T | 30.43 | | |

Table 10:

| Parameters / Test Solution | Relative biological activity (%) | | GCV value (n=12) |
|---|---|---|---|
| | Analyst 1 | Analyst 2 | |
| Test solution-1 | 111% | 96% | 38.70% |
| Test solution-2 | 127% | 101% | |
| Test solution-3 | 105% | 124% | |
| Test solution-4 | 92% | 76% | |
| Test solution-5 | 104% | 112% | |
| Test solution-6 | 116% | 97% | |

From the above analysis, it can be seen that when the HTRF kit method is used to detect glargine insulin injection, the precision GCV is greater, indicating that compared with the reporter gene analysis method of the present invention, the HTRF kit method has greater variability.

3.4 Comparison of Linearity and Accuracy

Two different analysts prepared linear solutions (also known as accuracy solutions) of insulin glargine with potency levels of 50%, 75%, 100%, 125%, and 150% on different days and tested them according to the HTRF kit method. The statistical results are shown in Table 11. The spike recovery rate of the HTRF kit method fluctuated more significantly. Statistical analysis revealed that the luciferase reporter gene analysis method developed in the present invention for detecting the biological activity of insulin glargine injection was close to the theoretical value (correlation 0.981, Figure 7).

Table 11:

| Theoretical relative biological activity | No. | Actual measured relative bioactivity (%) | Actual measured relative bioactivity mean (%) | Recovery rate (%) | Recovery rate mean (%) |
|---|---|---|---|---|---|
| 50% | 1 | 42 | 52 | 84 | 105 |
| | 2 | 60 | | 121 | |
| | 3 | 55 | | 110 | |
| 75% | 1 | 79 | 80 | 106 | 106 |
| | 2 | 81 | | 108 | |
| | 3 | 79 | | 105 | |
| 100% | 1 | 111 | 105 | 111 | 105 |
| | 2 | 99 | | 99 | |
| | 3 | 105 | | 105 | |
| 125% | 1 | 126 | 115 | 101 | 92 |
| | 2 | 97 | | 77 | |
| | 3 | 123 | | 99 | |
| 150% | 1 | 152 | 145 | 101 | 96 |
| | 2 | 156 | | 104 | |
| | 3 | 126 | | 84 | |

**Example 4: Application of biological activity detection of insulin or analogs thereof**

4.1 Detection of Ultra-Long-Acting Insulin

According to the embodiment of the present invention, a gradient dilution of a compound of ultra-long-acting insulin in a dosage range of 115.6 nM~10,000 nM was used, and the activity of ultra-long-acting insulin (A14E, B16H, B25H, B29K (N($\epsilon$)-COOH $(CH_2)_{16}$CO-$L$-Lys-CO $(CH_2)_2$CO-$(OEG)_2$), desB30 human insulin analogue) was detected using the method of the present invention (see the specific embodiments section for details).

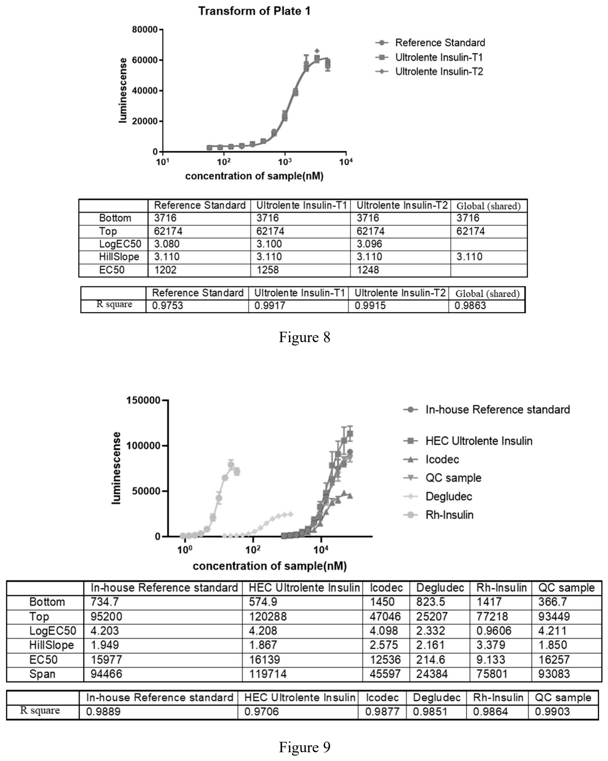

The results are shown in Figure 8. The detection method of the present invention was used to detect ultra-long-acting insulin. It was found that the reporter gene bioactivity analysis method has good applicability to it (ultra-long-acting insulin), good dose-effect relationship, and high repeatability. It can be used for in vitro bioactivity detection and research of ultra-long-acting insulin.

The results are shown in Figure 9. The detection method of the present invention was used to conduct in vitro efficacy studies on HEC's self-developed ultra-long-acting insulin molecules. By comparing the biological activities of recombinant human insulin, icodec, and degludec insulin after IRB phosphorylation, it was verified that the in vitro efficacy of HEC's self-developed ultra-long-acting insulin molecules met the design purpose. The results of this method showed that the biological activity of HEC's self-developed ultra-long-acting insulin molecules after IRB phosphorylation was equivalent to that of icodec, significantly lower than that of degludec insulin, and even more significantly lower than that of recombinant human insulin after IRB phosphorylation, which is consistent with the theoretical value, indicating that the present invention can accurately reflect the in vitro biological activity of insulin analog molecules with different design purposes, and can be used for in vitro mechanism studies of insulin analogs and screening of new insulin analog molecules.

4.2 Degludec and Aspartame Combination

According to the embodiment of the present invention, a gradient dilution of the compound of the degludec and aspartate combination preparation with a dilution factor ranging from 1~256 was used to detect the activity of the degludec and aspartate combination preparation using the method of the present invention (see the detailed embodiment section for details).

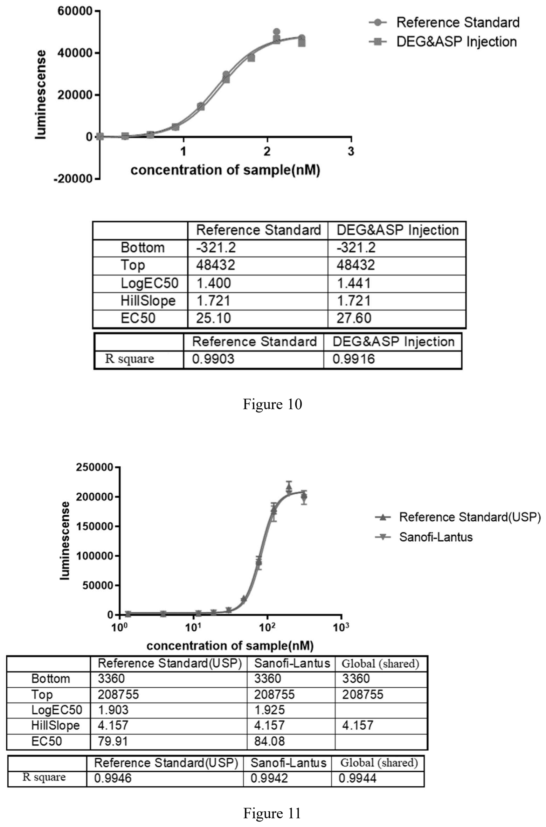

The results are shown in Figure 10. The detection method of the present invention was used to detect the degludec and aspartate compound preparation. It was found that the reporter gene bioactivity analysis method has good applicability to it (degludec and aspartate compound preparation), good dose-effect relationship, and high repeatability, and can be used for in vitro bioactivity detection and research of degludec and aspartate compound preparation.

4.3 Activity Testing of Insulin Glargine Injection (Sanofi)

According to the embodiment of the present invention, the activity of insulin glargine injection produced by Sanofi was detected using the method for detecting insulin or its analogs described in the present invention. For details of the specific steps, please refer to the specific implementation method (part on the method for detecting the activity of insulin or analogs thereof).

The results showed that the relative biological activity of insulin glargine injection (Sanofi) detected by the method of the present invention was 105% (Figure 11).

4.4 Activity Testing of Insulin Glargine Injection (HEC)

According to the embodiment of the present invention, the activity of insulin glargine injection produced by HEC was detected using the method for detecting insulin or its analogs described in the present invention. For details of the specific steps, please refer to the specific implementation method (part on the method for detecting the activity of insulin or analogs thereof).

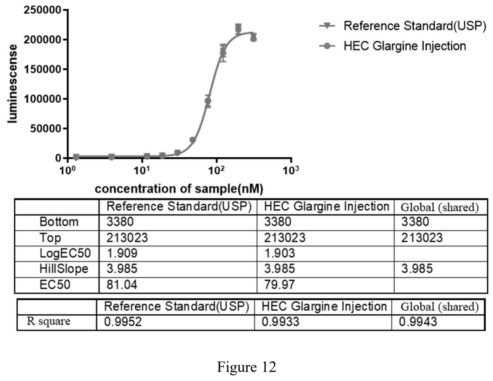

The results showed that the relative biological activity of insulin glargine injection (HEC) detected by the method of the present invention was 105% (Figure 12).

In summary, as shown in the above examples, the method for detecting the activity of insulin or its analogs designed by the present invention can be widely used.

Reference throughout this specification to "an embodiment," "some embodiments," "one embodiment", "another example," "an example," "a specific examples," or "some examples," means that a particular feature, structure, material, or characteristic described in connection with the embodiment or example is included in at least one embodiment or example of the present disclosure. In this specification, the schematic expressions of the above terms do not necessarily refer to the same embodiment or example. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

**Claims**

1.    An isolated nucleic acid molecule, **characterized in that** it comprises:

a first nucleic acid segment encoding insulin receptor B;
a second nucleic acid segment encoding a STAT5b response element;
a third nucleic acid segment encoding a reporter gene;
the STAT5b response element comprises the nucleic acid sequences as shown in any one of SEQ ID NO: 1~3.
the first nucleic acid segment, the second nucleic acid segment, and the second and third nucleic acid segments are linked or unlinked.

2.    The nucleic acid molecule according to claim 1, **characterized in that** the second nucleic acid fragment is linked to the third nucleic acid fragment.

3.    The nucleic acid molecule according to claim 1, **characterized in that** the STAT5b response element comprises the nucleic acid sequence as shown in SEQ ID NO:3.

4.    The nucleic acid molecule according to claim 1, **characterized in that** the reporter gene comprises at least one of a Lucifererase gene, a CAT gene, a YFP gene or a GFP gene;
preferably, the reporter gene is a Lucifererase gene.

5.    The nucleic acid molecule according to claim 1, **characterized in that** the insulin receptor protein B comprises the amino acid sequence as shown in SEQ ID NO: 4;
optionally, the reporter gene comprises the amino acid sequence as shown in SEQ ID NO: 5.

6. The nucleic acid molecule according to claim 1, **characterized in that** the first nucleic acid fragment comprises the nucleotide sequence as shown in SEQ ID NO: 6;
optionally, the third nucleic acid fragment comprises the nucleotide sequence as shown in SEQ ID NO: 7.

7. An expression vector, **characterized in that** it carries any nucleic acid fragment of claim 1 or the nucleic acid molecule of any one of claims 1 ~ 6.

8. The expression vector according to claim 7, **characterized in that** the expression vector is a non-pathogenic viral vector;
optionally, the non-pathogenic virus is selected from at least one of a retrovirus, a lentivirus and an adenovirus-associated virus.

9. A recombinant cell, **characterized in that** it carries the nucleic acid molecule of any one of claims 1 ~ 6 or the expression vector of any one of claims 7 ~ 8;

   optionally, the recombinant cell is selected from at least one of CRFK, HEK293FT, HEK293T, HEK293 or BHK cells;
   preferably, the recombinant cell is a HEK293 cell.

10. A method for detecting biological activity of insulin or an analog thereof, **characterized in that** it comprises:

    contacting a test sample of insulin or an analog thereof with the recombinant cell of claim 9;
    subjecting the product of said contacting to luminescence signal detection; and calculating the biological activity of insulin or an analog thereof based on the luminescence signal.

11. The method according to claim 10, **characterized in that**, after the contact treatment and before the detection treatment, an incubation treatment is further included, the incubation treatment is incubating the contacted product in a 37 °C, 5% $CO_2$ incubator for 10-20 h, preferably, incubator for 15 h;
optionally, the volume of the sample to be tested is 18-24 $\mu$l, preferably 20 $\mu$l.

12. A kit, **characterized in that** the kit comprises: the nucleic acid molecule of any one of claims 1 ~ 6, the expression vector of any one of claims 7 ~ 8, or the recombinant cell of claim 9.

13. A kit according to claim 12, **characterized in that**, the kit further includes an activity curve or instructions of a standard substance of insulin or analogs thereof.

14. Use of the nucleic acid molecule of any one of claims 1 ~ 6, the expression vector of any one of claims 7 ~ 8, the recombinant cell of claim 9, or the kit of any one of claims 12 ~ 13 for detecting the biological activity of insulin or an analog thereof.

15. The nucleic acid molecule of any one of claims 1 ~ 6, the expression vector of any one of claims 7 ~ 8, the recombinant cell of claim 9, or the kit of any one of claims 12 ~ 13 is used for detecting the biological activity of insulin or an analog thereof.

Figure 1

| | STAT5b-01 | STAT5b-02 | STAT5b-03 |
|---|---|---|---|
| Bottom | 10957 | 2239 | 1423 |
| Top | 19760 | 78073 | 129211 |
| LogEC50 | 1.131 | 1.431 | 1.278 |
| HillSlope | 0.6015 | 3.146 | 2.729 |
| EC50 | 13.51 | 26.96 | 18.95 |

| | STAT5b-01 | STAT5b-02 | STAT5b-03 |
|---|---|---|---|
| R square | 0.8597 | 0.9971 | 0.9926 |

Figure 2

## Transform of Data 1

| | STAT5b-02-Luciferase#001 | STAT5b-02-Luciferase#002 | STAT5b-03-Luciferase#001 | STAT5b-03-Luciferase#002 | STAT5b-02-Luciferase#003 | STAT5b-03-Luciferase#003 |
|---|---|---|---|---|---|---|
| Bottom | 6838 | 7695 | 6244 | 2835 | 3856 | 1965 |
| Top | 216285 | 171787 | 704554 | 72167 | 104630 | 121240 |
| LogEC50 | 1.037 | 1.175 | 0.8561 | 0.9565 | 0.9862 | 1.344 |
| HillSlope | 2.3 | 2.432 | 2.062 | 2.231 | 2.413 | 2.011 |
| EC50 | 10.89 | 14.97 | 7.18 | 9.047 | 9.686 | 22.07 |

| | STAT5b-02-Luciferase#001 | STAT5b-02-Luciferase#002 | STAT5b-03-Luciferase#001 | STAT5b-03-Luciferase#002 | STAT5b-02-Luciferase#003 | STAT5b-03-Luciferase#003 |
|---|---|---|---|---|---|---|
| R square | 0.9833 | 0.9781 | 0.9945 | 0.9868 | 0.9878 | 0.9951 |

Figure 3

## Specificity

| | Liraglutide | Glargine DP matrix | HTD Glargine DP | Reference Standard |
|---|---|---|---|---|
| Bottom | | 912 | 1930 | 1938 |
| Top | | 1380 | 103274 | 106372 |
| LogEC50 | | 1.511 | 1.767 | 1.652 |
| HillSlope | | 2.117 | 3.532 | 3.621 |
| EC50 | | 32.43 | 58.48 | 44.91 |

| | Liraglutide | Glargine DP matrix | HTD Glargine DP | Reference Standard |
|---|---|---|---|---|
| R square | | 0.4419 | 0.9926 | 0.9897 |

Figure 4

$y = 0.9619x + 0.0468$
$R^2 = 0.9959$

Figure 5

## Specificity

Legend:
- Glargine DS
- Glargine DP
- HTD(high-temperature destructive) Glargine DP
- Glargine DP matrix
- 0.01M HCL
- Liraglutide

|  | Glargine DS | Glargine DP | HTD Glargine DP |
|---|---|---|---|
| Bottom | 1872 | 1969 | 2159 |
| Top | 10195 | 10472 | 11660 |
| LogEC50 | 1.306 | 1.234 | 1.416 |
| HillSlope | 0.8654 | 0.9159 | 0.9136 |
| EC50 | 20.24 | 17.13 | 26.07 |

|  | Glargine DS | Glargine DP | HTD Glargine DP |
|---|---|---|---|
| R square | 0.9523 | 0.9701 | 0.9800 |

Figure 6

$$y = 0.884 x + 0.110$$
$$R^2 = 0.981$$

- ♦ Series 1
- — Linearity (Series 1)

Figure 7

**Transform of Plate 1**

| | Reference Standard | Ultrolente Insulin-T1 | Ultrolente Insulin-T2 | Global (shared) |
|---|---|---|---|---|
| Bottom | 3716 | 3716 | 3716 | 3716 |
| Top | 62174 | 62174 | 62174 | 62174 |
| LogEC50 | 3.080 | 3.100 | 3.096 | |
| HillSlope | 3.110 | 3.110 | 3.110 | 3.110 |
| EC50 | 1202 | 1258 | 1248 | |

| | Reference Standard | Ultrolente Insulin-T1 | Ultrolente Insulin-T2 | Global (shared) |
|---|---|---|---|---|
| R square | 0.9753 | 0.9917 | 0.9915 | 0.9863 |

Figure 8

| | In-house Reference standard | HEC Ultrolente Insulin | Icodec | Degludec | Rh-Insulin | QC sample |
|---|---|---|---|---|---|---|
| Bottom | 734.7 | 574.9 | 1450 | 823.5 | 1417 | 366.7 |
| Top | 95200 | 120288 | 47046 | 25207 | 77218 | 93449 |
| LogEC50 | 4.203 | 4.208 | 4.098 | 2.332 | 0.9606 | 4.211 |
| HillSlope | 1.949 | 1.867 | 2.575 | 2.161 | 3.379 | 1.850 |
| EC50 | 15977 | 16139 | 12536 | 214.6 | 9.133 | 16257 |
| Span | 94466 | 119714 | 45597 | 24384 | 75801 | 93083 |

| | In-house Reference standard | HEC Ultrolente Insulin | Icodec | Degludec | Rh-Insulin | QC sample |
|---|---|---|---|---|---|---|
| R square | 0.9889 | 0.9706 | 0.9877 | 0.9851 | 0.9864 | 0.9903 |

Figure 9

|  | Reference Standard | DEG&ASP Injection |
|---|---|---|
| Bottom | -321.2 | -321.2 |
| Top | 48432 | 48432 |
| LogEC50 | 1.400 | 1.441 |
| HillSlope | 1.721 | 1.721 |
| EC50 | 25.10 | 27.60 |

|  | Reference Standard | DEG&ASP Injection |
|---|---|---|
| R square | 0.9903 | 0.9916 |

Figure 10

|  | Reference Standard(USP) | Sanofi-Lantus | Global (shared) |
|---|---|---|---|
| Bottom | 3360 | 3360 | 3360 |
| Top | 208755 | 208755 | 208755 |
| LogEC50 | 1.903 | 1.925 | |
| HillSlope | 4.157 | 4.157 | 4.157 |
| EC50 | 79.91 | 84.08 | |

|  | Reference Standard(USP) | Sanofi-Lantus | Global (shared) |
|---|---|---|---|
| R square | 0.9946 | 0.9942 | 0.9944 |

Figure 11

|  | Reference Standard(USP) | HEC Glargine Injection | Global (shared) |
|---|---|---|---|
| Bottom | 3380 | 3380 | 3380 |
| Top | 213023 | 213023 | 213023 |
| LogEC50 | 1.909 | 1.903 |  |
| HillSlope | 3.985 | 3.985 | 3.985 |
| EC50 | 81.04 | 79.97 |  |

|  | Reference Standard(USP) | HEC Glargine Injection | Global (shared) |
|---|---|---|---|
| R square | 0.9952 | 0.9933 | 0.9943 |

Figure 12

## INTERNATIONAL SEARCH REPORT

| International application No. |
| --- |
| **PCT/CN2024/093792** |

| A. | CLASSIFICATION OF SUBJECT MATTER |
| --- | --- |

C07K14/62(2006.01)i;  C12N15/17(2006.01)i;  A61K38/28(2006.01)i;  A61P3/10(2006.01)i

According to International Patent Classification (IPC) or to both national classification and IPC

| B. | FIELDS SEARCHED |
| --- | --- |

Minimum documentation searched (classification system followed by classification symbols)

IPC:C07K,C12N,A61K,A61P

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, JPTXT, USTXT, EPTXT, 万方, WANFANG, CNKI, PubMed, GenBank, ISI web of knowledge, 中国专利序列数据库, China Patents Sequence Database: 胰岛素, 类似物, 胰岛素受体B, STAT5b响应元件, 反转录病毒, Insulin, Analogs, Insulin Receptor B, STAT5b Responsive Elements, Retroviruses

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | CN 101200707 A (XIHUA UNIVERSITY) 18 June 2008 (2008-06-18) claims 1-9, and description, embodiments | 1-15 |
| A | CN 114720703 A (TONGHUA DONGBAO PHARMACEUTICAL CO., LTD.) 08 July 2022 (2022-07-08) entire document | 1-15 |
| A | CN 106636154 A (SHANGHAI INSTITUTES FOR BIOLOGICAL SCIENCES, CHINESE ACADEMY OF SCIENCES) 10 May 2017 (2017-05-10) entire document | 1-15 |
| A | WO 2020123269 A1 (MERCK SHARP & DOHME CORP.) 18 June 2020 (2020-06-18) entire document | 1-15 |
| A | US 5863724 A (BAYLOR COLLEGE OF MEDICINE et al.) 26 January 1999 (1999-01-26) entire document | 1-15 |
| A | CN 114591417 A (SICHUAN UNIVERSITY) 07 June 2022 (2022-06-07) entire document | 1-15 |

☑ Further documents are listed in the continuation of Box C.　　☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **02 August 2024** | **09 August 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/093792** |

### C.    DOCUMENTS CONSIDERED TO BE RELEVANT

| Category* | Citation of document, with indication, where appropriate, of the relevant passages | Relevant to claim No. |
| --- | --- | --- |
| A | CN 111094572 A (JIANGSU HENGRUI PHARMACEUTICALS CO., LTD.) 01 May 2020 (2020-05-01)<br>     entire document | 1-15 |
| PX | CN 116751817 A (GUANGDONG HEC PHARMACEUTICAL CO., LTD.) 15 September 2023 (2023-09-15)<br>     claims 1-13, and description, specific embodiments | 1-15 |

Form PCT/ISA/210 (second sheet) (July 2022)

**INTERNATIONAL SEARCH REPORT**

International application No.

**PCT/CN2024/093792**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.   ☑  forming part of the international application as filed.

    b.   ☐  furnished subsequent to the international filing date for the purposes of international search (Rule 13*ter*.1(a)),

          ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/CN2024/093792**

| Patent document cited in search report | | | Publication date (day/month/year) | Patent family member(s) | | | Publication date (day/month/year) |
|---|---|---|---|---|---|---|---|
| CN | 101200707 | A | 18 June 2008 | None | | | |
| CN | 114720703 | A | 08 July 2022 | None | | | |
| CN | 106636154 | A | 10 May 2017 | None | | | |
| WO | 2020123269 | A1 | 18 June 2020 | US | 2022026414 | A1 | 27 January 2022 |
| | | | | WO | 2020123269 | A8 | 24 December 2020 |
| | | | | EP | 3894066 | A1 | 20 October 2021 |
| | | | | EP | 3894066 | A4 | 07 September 2022 |
| US | 5863724 | A | 26 January 1999 | US | 6054313 | A | 25 April 2000 |
| | | | | CA | 2187945 | A1 | 26 October 1995 |
| | | | | JPH | 09512166 | A | 09 December 1997 |
| | | | | WO | 9528411 | A1 | 26 October 1995 |
| | | | | EP | 0789705 | A1 | 20 August 1997 |
| | | | | EP | 0789705 | A4 | 28 April 1999 |
| | | | | AU | 2285595 | A | 10 November 1995 |
| | | | | AU | 694507 | B2 | 23 July 1998 |
| | | | | US | 6031150 | A | 29 February 2000 |
| CN | 114591417 | A | 07 June 2022 | None | | | |
| CN | 111094572 | A | 01 May 2020 | AU | 2019218315 | A1 | 09 July 2020 |
| | | | | EP | 3750998 | A1 | 16 December 2020 |
| | | | | EP | 3750998 | A4 | 23 February 2022 |
| | | | | US | 2021032307 | A1 | 04 February 2021 |
| | | | | CA | 3086618 | A1 | 15 August 2019 |
| | | | | TW | 201934752 | A | 01 September 2019 |
| | | | | MX | 2020007929 | A | 01 October 2020 |
| | | | | BR | 112020015238 | A2 | 29 December 2020 |
| | | | | KR | 20200119237 | A | 19 October 2020 |
| | | | | RU | 2020128190 | A3 | 09 March 2022 |
| | | | | JP | 2021513330 | A | 27 May 2021 |
| | | | | WO | 2019154311 | A1 | 15 August 2019 |
| CN | 116751817 | A | 15 September 2023 | None | | | |

Form PCT/ISA/210 (patent family annex) (July 2022)

**REFERENCES CITED IN THE DESCRIPTION**

**Patent documents cited in the description**

- CN 202310620396